(19) Europäisches Patentamt
European Patent Office
Office européen des brevets

(11) EP 0 946 679 B1

(12) **EUROPÄISCHE PATENTSCHRIFT**

(45) Veröffentlichungstag und Bekanntmachung des Hinweises auf die Patenterteilung:
**03.05.2000 Patentblatt 2000/18**

(21) Anmeldenummer: **97910422.1**

(22) Anmeldetag: **08.10.1997**

(51) Int Cl.[7]: **C10G 75/04**, E21B 37/06, C10L 1/22, C10L 1/14, C10L 10/00

(86) Internationale Anmeldenummer:
**PCT/EP97/05514**

(87) Internationale Veröffentlichungsnummer:
**WO 98/16595 (23.04.1998 Gazette 1998/16)**

# (54) VERWENDUNG VON SARKOSINATEN ALS ASPHALTEN-DISPERGATOREN

USE OF SARCOSINATES AS ASPHALTENE DISPERSANTS

UTILISATION DE SARCOSINATES COMME DISPERSANTS D'ASPHALTENE

(84) Benannte Vertragsstaaten:
**DE FR GB IT NL**

(30) Priorität: **15.10.1996 DE 19642494**

(43) Veröffentlichungstag der Anmeldung:
**06.10.1999 Patentblatt 1999/40**

(73) Patentinhaber: **Clariant GmbH**
**65929 Frankfurt am Main (DE)**

(72) Erfinder:
- **MILLER, Dennis**
  **D-65779 Kelkheim (DE)**
- **VOLLMER, Axel**
  **D-65830 Kriftel (DE)**
- **FEUSTEL, Michael**
  **D-55278 Köngernheim (DE)**
- **KLUG, Peter**
  **D-63762 Gro ostheim (DE)**

(56) Entgegenhaltungen:
**WO-A-88/07408**
**DE-A- 1 545 248**
**DE-A- 1 545 298**
**FR-A- 1 212 585**
**FR-A- 1 214 872**
**GB-A- 951 138**
**GB-A- 1 085 169**
**US-A- 4 614 236**
**US-A- 4 737 296**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 946 679 B1

Printed by Jouve, 75001 PARIS (FR)

## Beschreibung

Asphaltene sind Bestandteile von Rohölen. Sie enthalten eine Vielzahl von Strukturen, besonders hochmolekulare kondensierte aromatische Komponenten mit Heteroatomen. Angesichts der Komplexität ihrer Chemie werden Asphaltene als die Ölfraktion beschrieben, die in Benzol, aber nicht in n-Pentan löslich ist.

Im Rohöl liegen Asphaltene normalerweise als kolloidale Dispersion vor. Diese wird durch Ölharze stabilisiert.

Asphaltene können während der Produktion, der Raffination, des Transports und der Lagerung von Rohöl und davon abgeleiteten Produkten,wie z.B. schweres Heizöl oder Schiffsöl, ausfallen. Gemeinsame Ursachen für dieses Ausfallen sind ein Absinken der Temperatur oder ein Wechsel in der Zusammensetzung (z.B. Verdampfung von leicht flüchtigen Bestandteilen). Asphaltene können auch beim Fließen durch poröse Medien ausfallen. Fluten mit $CO_2$ während des Förderprozesses kann Asphaltene zum Flokkulieren oder zum Ausfallen bringen.

Manche Öle enthalten Kohlenwasserstoffwachse, die bei niedrigen Temperaturen ausfallen. Wechselwirkungen zwischen dem Ausfallen von Wachs und Asphaltenen können die Gesamtmenge an ausgefallener Substanz oder deren Bildungsgeschwindigkeit erhöhen.

Ausgefallene Asphaltene verursachen Probleme bei der Produktion und bei der Verarbeitung von Rohölen. Asphaltene schlagen sich nieder in Ventilen, Rohren und Fördereinrichtungen. An heißen Oberflächen, wie beispielsweise Wärmetauschern, kann die Carbonisierung dieser Niederschläge ihre Entfernung sehr schwierig machen. Die Niederschläge reduzieren den Wirkungsgrad von Anlagen und können im schlimmsten Fall zu einer kompletten Blokkierung und zu einem Produktionsstop führen, was hohe Kosten verursacht.

Schweröle, die oft zum Antrieb von Schiffen verwendet werden, enthalten beträchtliche Mengen an Asphaltenen. Das Ausfallen von Asphaltenen kann sowohl zu schlechter Verbrennung als auch zu Schwierigkeiten bei der Handhabung und bei der Lagerung des Treibstoffes führen. Auch bei mit schweren Ölen betriebenen Kraftwerken werden Verbrennungsstörungen durch Ausfällung von Asphaltenen beobachtet.

Bitumen, Schweröle und Rückstände werden manchmal mit Lösemittel verdünnt, um die Viskosität für den Transport zu reduzieren. Wenn dabei Asphaltene ausfallen, so ergeben sich damit Probleme bei der Handhabung.

Das Ausfallen von Asphaltenen kann durch kleine Mengen an Dispergatoren verhindert oder verringert werden. Diese Substanzen zeigen einen oder mehrere der folgenden Effekte:

a) Die Menge an Niederschlag wird reduziert;
b) der Niederschlag bildet sich langsamer;
c) der Niederschlag ist feiner verteilt; und
d) die Neigung des Niederschlages, sich auf Oberflächen abzulagern, wird reduziert.

Wenn sich bereits Niederschläge an Asphaltenen gebildet haben, können sie durch den Gebrauch von Lösemitteln entfernt werden. Die Zugabe eines Dispergators kann die Wirksamkeit dieser Lösemittel verbessern.

Eine Vielzahl von Asphalten-Dispergatoren sind bereits bekannt. CA 2 029 465 und CA 2 075 749 beschreiben Alkylphenolformaldehydharze in Kombination mit hydrophilen-lipophilen Vinylpolymeren. Die Asphaltendispergierenden Eigenschaften von Dodecylbenzolsulfonsäure wurden beschrieben in US 4 414 035, außerdem durch D.-L. Chang und H.S. Fogler (SPE paper No. 25185, 1993) und durch M.N. Bouts et al. (J. pet. Technol. 47, 782-7, 1995).

Oxalkylierte Amine werden beschrieben in US 5 421 993.

Die bisher bekannten Dispergatoren können die durch das Ausfallen von Asphaltenen verursachten Probleme nur teilweise lösen. Da Öle in ihrer Zusammensetzung variieren, können einzelne Dispergatoren nur in einem beschränkten Bereich wirksam arbeiten. Manchmal haben sogar kleine Änderungen in der Ölzusammensetzung einen großen Effekt auf die Dispergiereigenschaften für Asphaltene. Deshalb sind in einigen Fällen die bekannten Dispergatoren nicht zufriedenstellend und zusätzliche Typen sind erforderlich.

Es bestand somit die Aufgabe, neue Asphalten-Dispergatoren zur Verfügung zu stellen, die die beschriebenen Nachteile der bisher bekannten Dispergatoren nicht aufweisen.

Überraschenderweise wurde gefunden, daß Sarkosinate der Formel (I)

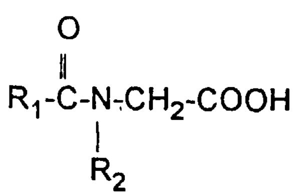

I

verwendet werden können, um das Ausfallen und/oder das Ablagern von Asphaltenen in Rohölen und davon abgeleiteten Produkten zu verhindern.

Gegenstand der Erfindung ist somit die Verwendung von Sarkosinaten der allgemeinen Formel (I)

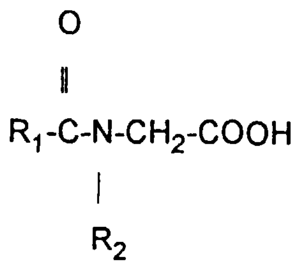

I

als Asphalten - Dispergator in Rohölen und davon abgeleiteten Produkte, worin

$R_1$ $C_7$-$C_{21}$-Alkyl oder Alkenyl, bevorzugt $C_{11}$-$C_{17}$, und
$R_2$ H oder $C_1$-$C_{22}$-Alkyl, bevorzugt H, Butyl oder Isobutyl bedeuten.

Die erfindungsgemäßen Dispergatoren werden in einer Konzentration von 1 bis 10.000 ppm, vorzugsweise von 2 bis 2.000 ppm eingesetzt.

Zur leichteren Dosierung können diese Asphalten-Dispergatoren als Lösung in einem ölmischbaren Lösemittel formuliert werden, wie beispielsweise aromatische Kohlenwasserstoffe oder Mischungen von Kohlenwasserstoffen und einem aliphatischen Alkohol.

Wirksame Asphalten-Dispergatoren werden ebenfalls erhalten durch Kombination dieser Sarkosinate mit anderen Komponenten, besonders

a) Alkylphenolformaldehydharze,
b) oxalkylierte Amine,
c) Alkylbenzolsulfonsäuren,
d) Wachsdispergatoren.

Dispergatoren, die auf einer Kombination von Substanzen basieren, können auf eine Änderung der Zusammensetzung des Öls weniger empfindlich sein; dies verbessert ihre Zuverlässigkeit.

Beispiele

Prinzip des Dispergiertestes

Die Dispergierung, das Ausfallen von Asphaltenen hängt von der Natur des Kohlenwasserstoffmediums ab. Asphaltene sind in aromatischen, aber nicht in aliphatischen Kohlenwasserstoffen löslich. Somit können Dispergatoren getestet werden, indem man das Öl oder extrahierte Asphaltene in einem aromatischen Lösemittel löst und indem man dann einen aliphatischen Kohlenwasserstoff zugibt, um einen Niederschlag zu erzeugen. Da Asphaltene von dunkler Farbe sind, kann das Ausmaß des Niederschlages durch eine kolorimetrische Messung der überstehenden Flüssigkeit bestimmt werden. Je dunkler die überstehende Flüssigkeit ist, desto mehr Asphaltene bleiben dispergiert, d.h. umso besser ist der Dispergator. Dieser Test wird beschrieben im Canadischen Patent 20 29 465. In unserer Version des Tests wird das Fällungsmedium ausgewählt, so daß die Asphaltene zum größten Teil, aber nicht komplett ausfallen.

Vorschrift Dispergiertest

a) Eine 25 %ige Öl-Lösung in Toluol wird gefiltert, um Verunreinigungen zu beseitigen;
b) 9,5 ml Heptan als Fällungsmittel für Asphaltene und 0,5 ml Toluol/Dispergator-Mischung (25:1) in ein gut 10 ml fassendes graduiertes Glasröhrchen vorlegen und gut schütteln. Dies entspricht einer Dispergatorkonzentration von 2000 ppm. Bei Bedarf kann die Menge Dispergator variiert werden. Für die Nullproben wird reines Toluol verwendet;
c) in das Glasröhrchen wird dann 0,1 ml von der gefilterten Öl-Lösung dazugegeben und ebenfalls gut geschüttelt;
d) das Ganze 2 Stunden ohne Erschütterungen stehenlassen. Die ausgefällten Asphaltene sollen sich am Boden

des Röhrchens sammeln können;

e) nach Ablauf dieser Zeit wird das Volumen des Sediments an Hand der Graduierung abgeschätzt, das Aussehen der gesamten Probe protokolliert und dann wird von der überständigen Phase 1 ml vorsichtig mit einer Pipette aufgenommen;

f) die abgesaugte Menge wird in 5 ml Toluol gelöst und bei 600 nm photometriert.

Bewertung des Dispergiertests

Als relatives Maß für die Dispergierwirkung wird folgender Ausdruck genommen

$$A = 100 (D - D_0)/D_0 ,$$

wobei D und $D_0$ optische Dichte von Meßlösung und Blindprobe sind. Der maximal erreichbare Wert von A, $A_{max}$, entspricht vollständiger Dispergierung der Asphaltene. Sie kann abgeschätzt werden, indem ein Versuch ohne Dispergator, mit Toluol anstatt Heptan, durchgeführt wird - dadurch bleiben die Asphaltene vollständig dispergiert.

Das Volumen des Sediments liefert eine weitere Information über die Wirksamkeit des Dispergators. Je kleiner die Menge an Sediment ist, desto besser dispergiert ist die Substanz.

Ergebnisse

Die Untersuchungen wurden ausgeführt mit einem Schweröl, das beträchtliche Mengen an Asphaltenen enthielt. Tabellen 1 und 2 zeigen die Ergebnisse des Dispergiertests mit verschiedenen Substanzen gemäß der Erfindung. Die Substanzen 2-6 wurden nach der in EP-A-0 680 948 offenbarten Methode hergestellt.

Tabelle 1 -

| getestete Substanzen | | | |
|---|---|---|---|
| Substanz Nr. | Chemie | | |
| | $R_1$ | $R_2$ | Beschreibung |
| 1 | $C_{17}H_{33}$ | H | Oleylsarcosin |
| 2 | i-$C_8H_{17}$ | n-$C_4H_9$ | Isononanoyl-n-butylglycin |
| 3 | $C_{15/17}H_{31/35}$ | i-$C_4H_9$ | Talgfettsäure-iso-butylglycin |
| 4 | $C_{11/13}H_{23/27}$ | $C_{12/14}H_{25/29}$ | Cocoyl-cocoyl-glycin |
| 5 | $C_{11/13}H_{23/27}$ | n-$C_4H_9$ | Cocoyl-n-butylglycin |
| 6 | $C_{15/17}H_{31/35}$ | n-$C_4H_9$ | Talgfettsäure-n-butylglycin |
| 7 | $C_{11/13}H_{23/27}$ | H | Lauroylsarcosin |

Tabelle 2 -

| Testergebnisse bei 2000 ppm Dispergator | | |
|---|---|---|
| Substanz Nr. | Dispergier-Effekt | |
| | A | Sediment (ml) |
| 1 | 334 | 0,3 |
| 2 | 238 | 0,6 |
| 3 | 270 | 0,5 |
| 4 | 323 | 0,3 |
| 5 | 241 | 0,5 |
| 6 | 247 | 0,6 |
| 7 | 290 | 0,4 |

Tabelle 2 - (fortgesetzt)

| Testergebnisse bei 2000 ppm Dispergator | | |
|---|---|---|
| Substanz Nr. | Dispergier-Effekt | |
| | A | Sediment (ml) |
| unbehandelt | 0 | 1,0 |

Unter diesen Bedingungen würde der maximale Wert für A, der einer kompletten Dispergierung des Asphaltens entspricht, bei etwa 500 liegen.

Die Dispergiereigenschaften der Substanz 1 konnten verbessert werden, indem man Nonylphenolformaldehydharz dazu mischte. So zeigte eine 1:1-Mischung dieser zwei Substanzen einen A-Wert von 475.

## Patentansprüche

1. Verwendung von Sarkosinaten der allgemeinen Formel (I)

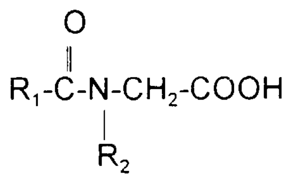

I

als Asphalten-Dispergator in Rohölen und davon abgeleiteten Produkten, worin

$R_1$ $C_7$-$C_{21}$-Alkyl oder -Alkenyl und
$R_2$ H oder $C_1$-$C_{22}$-Alkyl bedeuten.

2. Verwendung von Sarkosinaten gemäß Anspruch 1, dadurch gekennzeichnet, daß

$R_1$ $C_{11}$-$C_{17}$-Alkyl oder -Alkenyl und
$R_2$ H, Butyl oder Isobutyl bedeuten.

3. Verwendung von Sarkosinaten gemäß Anspruch 1 und/oder 2, dadurch gekennzeichnet, daß die Sarkosinate in einer Menge von 1 bis 10.000, vorzugsweise von 2 bis 2.000 ppm zugegeben werden.

4. Verwendung von Sarkosinaten gemäß einem oder mehreren der Ansprüche 1 bis 3, dadurch gekennzeichnet, daß zusätzlich Alkylphenolformaldehydharze, oxalkylierte Amine, Wachsdispergatoren oder beliebigen Mischungen daraus verwendet werden.

## Claims

1. The use of a sarcosinate of the formula (I)

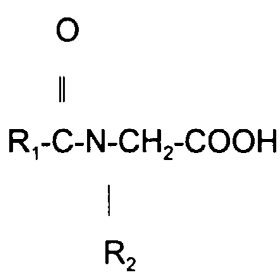

I

as asphaltene-dispersing agent in a crude oil or a product derived therefrom, in which

$R_1$ is $C_7$-$C_{21}$-alkyl or -alkenyl and
$R_2$ is H or $C_1$-$C_{22}$-alkyl.

**2.** The use of a sarcosinate as claimed in claim 1, wherein

$R_1$ is $C_{11}$-$C_{17}$-alkyl or -alkenyl and
$R_2$ is H, butyl or isobutyl.

**3.** The use of a sarcosinate as claimed in claim 1 and/or 2, wherein the sarcosinate is added in an amount of 1 to 10,000, preferably 2 to 2000 ppm.

**4.** The use of a sarcosinate as claimed in one or more of claims 1 to 3, wherein an alkylphenol-formaldehyde resin, oxalkylated amine, wax-dispersing agent or any desired mixture thereof is additionally used.

## Revendications

**1.** Utilisation de sarcosinates de formule générale (I)

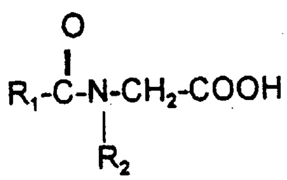

I

comme agent dispersant d'asphaltène dans les huiles brutes et les produits en dérivant, dans laquelle

$R_1$ représente un groupe alkyle ou alcényle en $C_7$ à $C_{21}$, et
$R_2$ représente un atome d'hydrogène ou un groupe alkyle en $C_1$ à $C_{22}$.

**2.** Utilisation de sarcosinates selon la revendication 1, caractérisée en ce que

$R_1$ représente un groupe alkyle ou alcényle en $C_{11}$ à $C_{17}$, et
$R_2$ représente un atome d'hydrogène, un groupe butyle ou isobutyle.

**3.** Utilisation de sarcosinates selon la revendication 1 et/ou 2, caractérisée en ce qu'on ajoute les sarcosinates en une quantité de 1 à 10 000, de préférence de 2 à 2 000 ppm.

**4.** Utilisation de sarcosinates selon une ou plusieurs des revendications 1 à 3, caractérisée en ce qu'on utilise en plus des résines alkylphénol-formaldéhyde, des amines oxalkylées, des dispersants de cire ou des mélanges quelconques de ceux-ci.